# FASCICULE DE BREVET EUROPEEN

(11) **EP 2 596 771 B1**
(45) Date de publication et mention de la délivrance du brevet: **28.10.2015**
(21) Numéro de dépôt: 12191300.8
(22) Date de dépôt: 05.11.2012
(51) Int. Cl.: A61F 5/02

(54) **Orthèse ajustable**
Einstellbare Orthese
Adjustable orthesis

(30) Priorité: 24.11.2011 FR 1160765
(43) Date de publication de la demande: 29.05.2013
(73) Titulaire: RICHARD FRERES, 42530 Saint Genest Lerpt (FR)
(72) Inventeur: Calet, Angélique, 42150 La Ricamarie (FR); Faure, Didier, 43140 Saint Didier en Velay (FR); Richard, Dominique, 42110 Aurec/Loire (FR)
(74) Mandataire: Delorme, Nicolas

(56) Documents cités:
- WO-A-2009/113053
- WO-A2-02/19954
- US-A- 5 387 183
- US-A1- 2002 052 568
- US-A1- 2009 093 745

## Description

La présente invention concerne une orthèse ajustable notamment destinée à une utilisation de ceinture lombaire.

Les orthèses conventionnelles servant de ceinture lombaire ont une fonction générale de maintien et de rappel de posture. Il est connu du document WO2009/113053 des dispositifs de soutien de la colonne vertébrale et bretelles configurés pour soutenir le bas du dos et, plus spécifiquement, des dispositifs d'ancrage sur des portions du bassin, configurés pour soutenir et / ou aligner une ou plusieurs vertèbres lombaires.

Il également connu du document US5387183 une ceinture multi-couches de support du dos et de l'abdomen ayant une couche primaire généralement élastique, une couche secondaire inflexible et non élastique, et une couche tertiaire de haute tension élastique. La ceinture est en outre pourvue d'attaches de coopération et d'éléments de fixation pour maintenir les éléments dans un engagement coopératif.

De façon classique les orthèses présentent des extrémités dotées d'éléments de fermeture réglable. Mais lorsque l'orthèse est destinée à s'ajuster sur des patients ayant des différences de taille significatives, l'orthèse perd de son efficacité. Les moyens de rappels de posture étant positionnés de façon fixe sur l'orthèse, l'orthèse ne peut pas être adaptée à toutes les tailles des patients. En effet, les moyens de rappels de posture peuvent être trop espacés pour être totalement efficaces sur un patient ayant une petite taille de ceinture, tandis que les moyens de rappels peuvent être trop rapprochés pour le maintien d'un patient de plus forte taille. De même, lorsque l'orthèse est utilisée par un patient ayant une petite taille de ceinture, la fermeture de l'orthèse nécessite de superposer les portions superflues de la ceinture lombaire. Cette superposition de matière rend alors difficile le port de l'orthèse sous les vêtements.

Un des buts de l'invention est de pallier à au moins l'un de ses inconvénients. A cet effet, l'invention a pour objet une orthèse présentant la forme d'une ceinture et comportant :
- une première bande comportant une portion de liaison dotée d'au moins un moyen de rappel de posture, des moyens de fixation et une portion abdominale disposant de moyens de fermeture ;
- une deuxième bande comportant une portion de liaison dotée d'au moins un moyen de rappel de posture, des moyens de fixation et une portion abdominale disposant de moyens de fermeture complémentaires adaptés pour coopérer de façon ajustable avec les moyens de fermeture ; et
- un panneau dorsal comportant
   o des moyens de fixation complémentaires agencés pour coopérer de façon ajustable avec les moyens de fixation de la première et de la deuxième bandes, et
   o deux moyens de rappel de posture destinés à être positionnés de part et d'autre de la colonne vertébrale d'un patient ;

les moyens de fixation de la première et de la deuxième bandes et les moyens de fixation complémentaires étant conformés de manière à autoriser un positionnement des moyens de rappel de posture de la première bande et de la deuxième bandes de part et d'autre des moyens de rappel de posture du panneau dorsal,

la première bande comprenant une languette comprenant des premiers moyens d'accroche et étant apte à être superposée au moins en partie à la portion de liaison de la deuxième bande comprenant des premiers moyens d'accroches complémentaires, les premiers moyens d'accroches complémentaires étant aptes à coopérer de manière ajustable avec les premiers moyens d'accroche, et la deuxième bande comprenant une languette comprenant des deuxièmes moyens d'accroche complémentaires et étant apte à être superposée au moins en partie à la portion de liaison de la première bande comprenant des deuxièmes moyens d'accroche, les deuxièmes moyens d'accroche complémentaires étant aptes à coopérer de manière ajustable avec les deuxièmes moyens d'accroche.

Grâce à cette configuration, le réglage de la taille de la ceinture peut se faire par un ajustement de la position de la première et de la deuxième bande de l'orthèse par rapport au panneau dorsal. Le moyen de rappel de posture de chaque bande peut alors être positionné de façon adaptée à la morphologie du patient. Ce type de réglage permet un ajustement de l'orthèse aux différentes tailles de ceinture des patients en assurant un gainage efficace. Cette orthèse peut alors remplacer plusieurs orthèses de taille unique. En complément, le réglage de la taille de la ceinture peut être affiné par la fixation ajustée des moyens de fermeture avec les moyens de fermeture complémentaires de la portion abdominale des bandes. Il est possible de pratiquer un ajustement sur quelques centimètres par exemple de sorte à ne pas déséquilibrer le positionnement des moyens de rappel de posture. Ainsi, l'orthèse selon l'invention est universelle en ce qu'elle est adaptée pour le gainage de tous les patients, quel que soit leur tour de ceinture. Par ailleurs, la configuration de cette orthèse permet aux moyens de rappel de posture de prendre appui entre les os du bassin et les vertèbres de la colonne vertébrale du patient de sorte à assurer un bon gainage dorsal et abdominal. De plus, l'orthèse de la présente invention est découpée en trois portions distinctes qui sont de ce fait plus faciles à stocker, notamment en officine.

Par ailleurs, il est également possible d'ajuster le positionnement des moyens de rappel de posture pour différents tours de taille tout en évitant la superposition d'une grande masse de matière et l'obtention d'une orthèse très épaisse. Il est alors possible de positionner l'orthèse sous les vêtements, directement sur la peau, ce qui rend le gainage invisible et évite le déplacement de l'orthèse avec le mouvement des vêtements.

La configuration des premiers moyens d'accroche et des premiers moyens d'accroche complémentaire permet de fixer les portions de liaisons des deux bandes entre elles ce qui peut ensuite faciliter la fixation des bandes sur le panneau dorsal.

La coopération entre les deuxièmes moyens d'accroche et deuxièmes moyens d'accroche complémentaires renforce l'intensité de la fixation des portions de liaison obtenue par l'assemblage des premiers moyens d'accroche aux premiers moyens d'accroches complémentaires.

Avantageusement, la portion abdominale de la première et de la deuxième bande comportent chacune un moyen de rappel de posture. L'orthèse peut ainsi gainer et maintenir la partie dorsale et la partie abdominale du patient en même temps.

Dans un mode de réalisation particulier, les bandes présentent une forme sensiblement symétrique, ce qui facilite leur fabrication.

Avantageusement, la portion de liaison de la première et de la deuxième bande et le panneau dorsal comprennent des références de positionnement agencées pour définir des positions d'ajustement prédéterminées dans lesquelles les moyens de rappel de posture de la première et de la deuxième bandes sont positionnés de façon symétrique par rapport aux moyens de rappel de posture du panneau dorsal. L'orthèse est alors facile à régler, facile à utiliser tout en garantissant le bon positionnement des moyens de rappel de posture.

Selon une variante, la première et la deuxième bande comprennent par exemple, chacune trois positions d'ajustements prédéterminées, de sorte à pouvoir s'ajuster à trois tours de tailles de patients. Ces tours de tailles peuvent par exemple à titre indicatif correspondre à des plages allant de 70 à 95 cm, de 95 à 110 cm et de 110 à 103 cm mais les tours de taille peuvent bien sûr correspondre à d'autres dimensions

Dans un mode de réalisation particulier de l'invention, les références de positionnement sont des coutures contrastantes disposées sur le panneau dorsal et sur les première et deuxième bandes.

Avantageusement, une partie centrale du panneau dorsal ou la totalité du panneau dorsal comporte une dimension axiale plus importante que celle des première et deuxième bandes. Cette configuration permet par exemple de maintenir les vertèbres lombaires d'un patient.

Dans une autre variante, les première et deuxième bandes sont adaptées pour être fixées à des panneaux dorsaux présentant des hauteurs différentes. Cette configuration est avantageuse en ce que la hauteur du panneau dorsal peut être choisie en fonction de la taille de l'orthèse. Il notamment été noté à titre d'exemple, que pour des tailles d'orthèses allant de 65 à 75 cm, 75 à 90 cm et 90 à 105 cm, un panneau dorsal d'une hauteur de 21 cm était particulièrement bien adapté. Egalement à titre d'exemple, pour des tailles d'orthèses allant de 90 à 105, de 105 à 120 cm et de 120 à 135 cm, un panneau dorsal d'une hauteur de 26 cm pouvait convenir.

Selon une variante, les moyens de fixation s'étendent sur la première et deuxième bande de sorte à recouvrir les positions des moyens de rappel de posture des portions de liaison de la première et de la deuxième bandes. De la sorte, les moyens de rappel de posture des portions de liaison sont efficacement retenus en place contre le panneau dorsal de l'orthèse. De plus, il est possible de fabriquer le fourreau de réception de moyen de rappel de posture et les moyens de fixation en une seule étape.

De préférence, les moyens de fixation complémentaires s'étendent selon la direction longitudinale de l'orthèse. Cette configuration facilite le réglage de la fixation des bandes pour différentes tailles des patients.

Selon une autre variante, les moyens de fixation de la première et de la deuxième bande s'étendent selon la direction longitudinale de l'orthèse de sorte à pouvoir ajuster la taille de l'orthèse.

Dans une alternative, les moyens de fixation ainsi que les moyens de fixation complémentaires s'étendent selon la direction longitudinale de l'orthèse.

Avantageusement, le moyen de rappel de posture est formé d'une baleine qui offre la rigidité attendue.

Dans un mode de réalisation particulier, les moyens de fixation, les moyens de fixation complémentaires, les moyens d'accroche, les moyens d'accroche complémentaire, les moyens de fermeture et les moyens de fermeture complémentaires comportent un textile de type boucles et crochets et dont l'assemblage offre une forte résistance à la tenue.

De préférence, le panneau dorsal, la première et la deuxième bandes comportent un matériau textile élastique.

L'invention sera mieux comprise à l'aide de la description qui suit en référence aux dessins schématiques annexés représentant un assemblage de deux demi-pièces à titre d'exemples non limitatifs. Dans la suite de la description, les éléments portant les mêmes références numériques sont identiques.
La figure 1 est une vue schématique d'une orthèse selon un mode de réalisation de l'invention.
La figure 2 est une vue schématique agrandie illustrant une portion de liaison d'une bande d'une orthèse selon un mode de réalisation de l'invention.
La figure 3 est une vue schématique d'un panneau dorsal d'une orthèse selon un mode de réalisation de l'invention.
La figure 4 est une vue schématique de deux bandes d'une orthèse selon un mode de réalisation de l'invention.
La figure 5 illustre une orthèse fermée selon un mode de réalisation de l'invention.

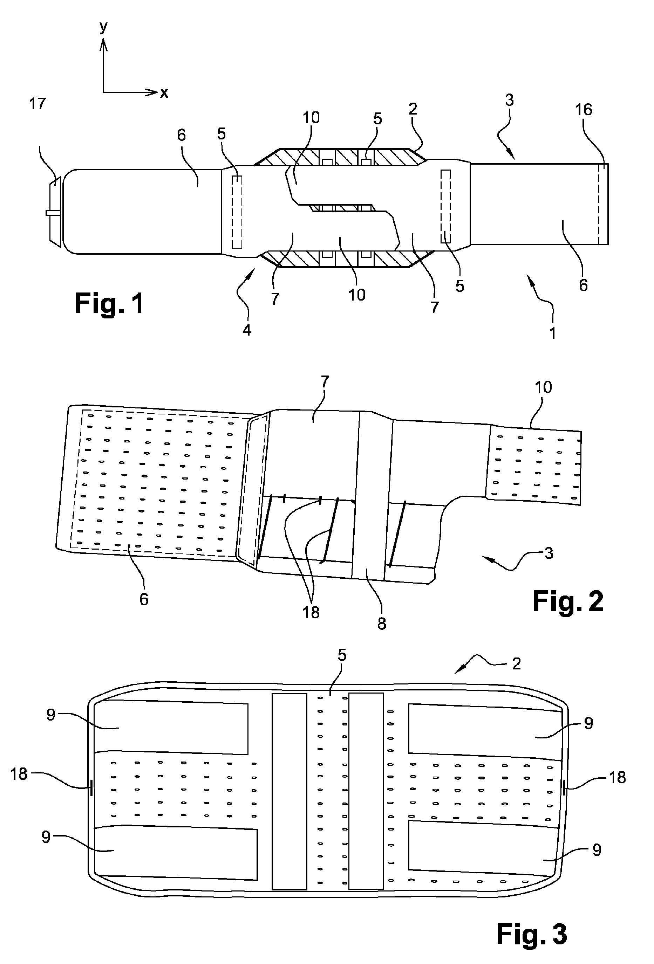

La figure 1 illustre une orthèse 1 en position ouverte présentant une forme de ceinture. L'orthèse 1 comprend un panneau dorsal 2, une première bande 3 et une deuxième bande 4 qui présentent une forme symétrique. Le panneau dorsal 2 comprend des moyens de rappel de posture 5, (également visibles à la figure 2), destinés à être placé de part et d'autre de la colonne vertébrale d'un patient. Ces moyens de rappel de posture 5 sont par exemple des baleines. De préférence, les baleines sont positionnées de façon symétrique à la colonne vertébrales afin d'assurer un gainage optimal.

Le panneau dorsal 2 présente une hauteur plus importante que celle des bandes 3,4, selon une direction axiale symbolisée par l'axe y de la figure 1, de sorte à obtenir un bon gainage des vertèbres lombaires. Dans un mode de réalisation non représenté, il est également possible que cette différence de hauteur ne soit prévue que sur une partie centrale du panneau 2 et/ou que d'un seul coté de l'axe longitudinal (symbolisé par l'axe x de la figure 1) du panneau dorsal 2.

Selon un mode de réalisation non illustré, il est également possible d'utiliser ces mêmes bandes avec des hauteurs différentes du panneau dorsal, augmentant ainsi le nombre de combinaisons et/ou de possibilités d'adaptation.

La première et la deuxième bandes 3,4 comprennent chacune une portion abdominale 6 et une portion de liaison 7 pour une fixation avec le panneau dorsal 2. Les portions de liaison 7 de chaque bande 3,4 comprennent chacune des moyens de fixation 8 au panneau dorsal 2 (figure 2). Le panneau dorsal 2 comprend des moyens de fixation complémentaires 9 aptes à coopérer de façon ajustable avec les moyens de fixation des deux bandes 3,4 de sorte que les baleines des bandes 3,4 soient situées de part et d'autre des baleines du panneau dorsal 2 (figure 3).

Les portions de liaisons des deux bandes 3,4 comprennent des moyens de rappel de posture 5 pouvant se présenter sous la forme de baleines, de sorte à compléter le gainage obtenu avec le panneau dorsal 2. Les fourreaux de réception des baleines présentent la même forme et sont placés au même endroit que les moyens de fixation 8 si bien qu'ils peuvent être confectionnés en même temps.

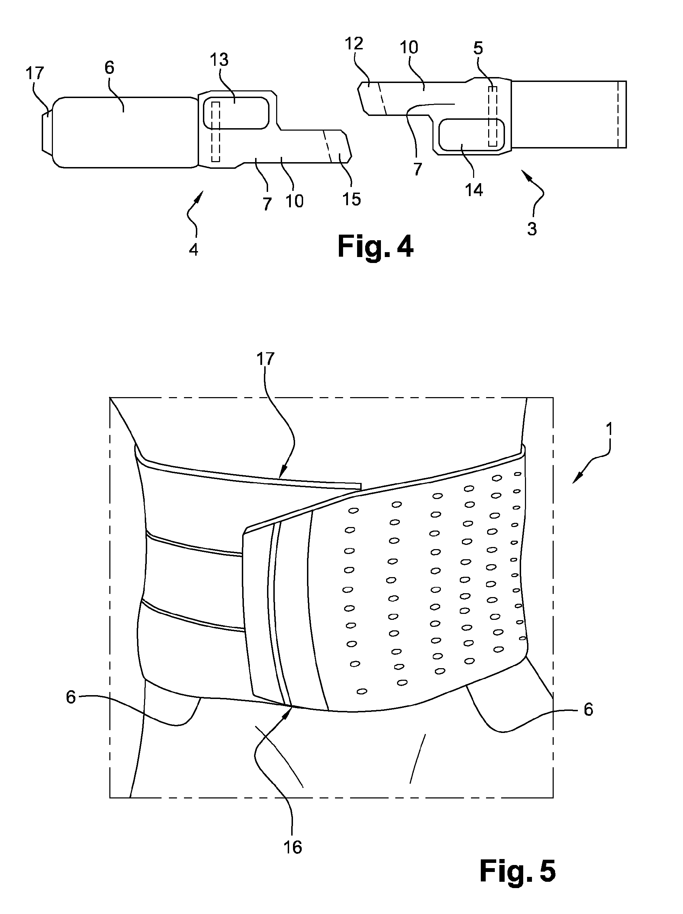

En référence à la figure 1, les portions de liaison 7 comprennent également chacune une languette 10. La languette 10 d'une première bande 3 étant conçue pour être superposée à la portion de liaison 7 d'une deuxième bande 4 et réciproquement. Des premiers moyens d'accroche 12 sont prévus sur la languette 10 de la première bande 3 (coté face verso illustrée à la figure 4) de sorte à coopérer de manière ajustable avec des premiers moyens d'accroche complémentaires 13 prévus sur la portion de liaison 7 de la face recto la deuxième bande 4. Cette conformation permet d'accrocher les portions de liaison 7 des deux bandes 3,4 entre elles avant de les fixer par le biais des moyens de fixation 8 au panneau dorsal 2. De plus, cette double coopération permet également de renforcer la fixation des différents éléments 2,3,4 constituant l'orthèse 1. Des deuxièmes moyens d'accroche 14 sont également prévus sur la languette 10 de la face recto de la première bande 3 de sorte à coopérer de manière ajustable avec des deuxièmes moyens d'accroche complémentaires 15 prévus sur la portion de liaison 7 de la face verso de la deuxième bande 4. Cette configuration permet d'éviter un recouvrement trop important des bandes 3,4 entre elles, tout en facilitant le réglage de la longueur de l'orthèse 1 à la taille du patient, avec une fixation renforcée des éléments 2,3,4 la constituant. Les moyens d'accroche 12,13,14,15 peuvent être constitués d'un matériau textile de type boucles et crochets.

En référence à la figure 1, la portion abdominale 6 de la première bande 3 comprend des moyens de fermeture 16 destiné à coopérer avec des moyens de fermeture complémentaires 17 de la portion abdominale 6 de la deuxième bande 4. Ces moyens de fermeture 16 sont par exemple un textile de type boucles et crochets. L'un des moyens de fermeture 16, par exemple en textile de type boucles, s'étend selon une direction longitudinale de la bande 3,4 sur une surface plus importante que celle du moyen de fermeture en textile de type crochets. Cette configuration permet de pouvoir ajuster le positionnement du textile complémentaire (dans cet exemple un textile de type crochets) selon la direction longitudinale de sorte à ajuster la dimension de l'orthèse 1 sur quelques centimètres, au niveau abdominal du patient. L'ajustement permis par ces moyens de fermeture 16,17 est prévu pour adapter plus finement l'orthèse 1 dans la plage de taille spécifiée ainsi que sa contention. Par ailleurs, dans le cas de petites tailles, la longueur des bandes 3,4 superflues et donc superposées serait trop importante pour que la ceinture puisse être portée sous les vêtements.

Selon une variante non représentée, la portion abdominale 6 de chaque bande 3,4 comprend un moyen de rappel de posture 5, telle qu'une baleine, de sorte que l'orthèse 1 puisse à la fois gainer le dos et l'abdomen d'un patient.

Comme illustré à la figure 2, les portions de liaison 7 comprennent également des références de positionnement 18 agencées pour définir des positions d'ajustement prédéterminées, telles que des coutures contrastantes, dans lesquelles les moyens de rappel de posture 5 de la première et de la deuxième bandes 3,4 sont positionnés de façon symétrique par rapport aux moyens de rappel de posture 5 du panneau dorsal 2.

En référence à la figure 3, des références de positionnement 18, telles que des coutures contrastantes, sont également prévues sur le panneau dorsal 2 de sorte à faciliter le positionnement 18 avec les portions de liaison 7. Les moyens de fixation complémentaires 9 s'étendent sur une portion importante du panneau 2 selon une direction longitudinale de l'orthèse 1 de sorte que les moyens de fixation 8 des bandes 3,4 puissent se fixer de façon réglable dans cette direction. Les moyens de fixation 8 et les moyens de fixation complémentaires 9 peuvent être obtenus par un textile de type boucles et crochets.

De la sorte, l'orthèse 1 peut ceinturer différents tours de taille sans engendrer une trop grande superposition de matériau. De plus, cette conformation permet aux baleines des bandes 3,4 d'être positionnées de façon optimale selon les morphologies des patients.

Dans un mode de réalisation non représenté, les moyens de fixation 8 des bandes 3,4 peuvent présenter une surface plus importante selon une direction longitudinale que celle des moyens de fixation complémentaires 9. Le réglage de la taille de l'orthèse 1 s'effectue de la même façon que dans la configuration précédente.

Le panneau dorsal 2, ainsi que les deux bandes 3,4 peuvent comporter différents éléments en matériau textile élastique de sorte à bien épouser les formes du corps du patient.

En référence à la figure 5, les moyens de fermeture 16 et moyens de fermetures complémentaires 17 sont ajustés pour fermer l'orthèse 1 autour du torse d'un patient.

## Revendications

1. Orthèse (1) présentant la forme d'une ceinture et comportant :
- une première bande (3) comportant une portion de liaison (7) dotée d'au moins un moyen de rappel de posture (5), des moyens de fixation (8) et une portion abdominale (6) disposant de moyens de fermeture (16) ;
- une deuxième bande (4) comportant une portion de liaison (7) dotée d'au moins un moyen de rappel de posture (5), des moyens de fixation (8) et une portion abdominale (6) disposant de moyens de fermeture complémentaires (17) adaptés pour coopérer de façon ajustable avec les moyens de fermeture (16); et
- un panneau dorsal (2) comportant
∘ des moyens de fixation complémentaires (9) agencés pour coopérer de façon ajustable avec les moyens de fixation (8) de la première et de la deuxième bandes (3,4), et
∘ deux moyens de rappel de posture (5) destinés à être positionnés
de part et d'autre de la colonne vertébrale d'un patient ;
les moyens de fixation (8) de la première et de la deuxième bandes (3,4) et les moyens de fixation complémentaires (9) étant conformés de manière à autoriser un positionnement des moyens de rappel de posture (5) de la première bande (3) et de la deuxième bande (4) de part et d'autre des moyens de rappel de posture (5) du panneau dorsal (2),
l'orthèse étant **caractérisée en ce que** la première bande (3) comprend une languette (10) comprenant des premiers moyens d'accroche (12) et étant apte à être superposée au moins en partie à la portion de liaison (7) de la deuxième bande (4) comprenant des premiers moyens d'accroches complémentaires (13), les premiers moyens d'accroches complémentaires (13) étant aptes à coopérer de manière ajustable avec les premiers moyens d'accroche (12), et **en ce que** la deuxième bande (4) comprend une languette (10) comprenant des deuxièmes moyens d'accroche complémentaires (15) et étant apte à être superposée au moins en partie à la portion de liaison (7) de la première bande (3) comprenant des deuxièmes moyens d'accroche (14), les deuxièmes moyens d'accroche complémentaires (15) étant aptes à coopérer de manière ajustable avec les deuxièmes moyens d'accroche (14).

2. Orthèse (1) selon la revendication 1, **caractérisée en ce que** les portions abdominales (6) de la première et de la deuxième bandes (3,4) comportent chacune un moyen de rappel de posture (5).

3. Orthèse (1) selon l'une des revendications 1 à 2, **caractérisée en ce que** les portions de liaison (7) de la première et de la deuxième bandes (3,4) et le panneau dorsal (2) comprennent des références de positionnement (18) agencées pour définir des positions d'ajustement prédéterminées dans lesquelles les moyens de rappel de posture (5) de la première et de la deuxième bandes (3,4) sont positionnés de façon symétrique par rapport aux moyens de rappel de posture (5) du panneau dorsal (2).

4. Orthèse (1) selon l'une des revendications 1 à 3, **caractérisée en ce que** les références de positionnement (18) sont des coutures contrastantes disposées sur le panneau dorsal (2) et sur la première et la deuxième bandes (3,4).

5. Orthèse (1) selon l'une des revendications 1 à 4, **caractérisée en ce qu'**une partie centrale du panneau dorsal (2) ou la totalité du panneau dorsal (2) comporte une dimension axiale plus importante que celle de la première et de la deuxième bandes (3,4).

6. Orthèse (1) selon l'une des revendications 1 à 5, **caractérisée en ce que** les moyens de fixation (8) s'étendent sur la première et la deuxième bande (3,4) de sorte à recouvrir les positions des moyens de rappel de posture (5) des portions de liaison (7) de la première et de la deuxième bandes (3,4).

7. Orthèse (1) selon l'une des revendications 1 à 6, **caractérisée en ce que** les moyens de fixation complémentaires (9) s'étendent selon la direction longitudinale de l'orthèse (1).

8. Orthèse (1) selon l'une des revendications 1 à 7, **caractérisée en ce que** le moyen de rappel de posture (5) est formé d'une baleine.

9. Orthèse (1) selon l'une des revendications 1 à 8, **caractérisée en ce que** les moyens de fixation (8), les moyens de fixation complémentaires (9), les moyens d'accroche (12,14), les moyens d'accroche complémentaire (13, 15), les moyens de fermeture (16) et les moyens de fermeture complémentaires (17) comportent un textile de type crochets ou boucles.

10. Orthèse (1) selon l'une des revendications 1 à 9, **caractérisée en ce que** le panneau dorsal (2), les première et deuxième bandes (3,4) comportent un matériau textile élastique.

## Patentansprüche

1. Orthese (1), die die Form eines Gürtels aufweist und Folgendes umfasst:
- ein erstes Band (3), das einen Verbindungsabschnitt (7), der mit mindestens einem Haltungsrückstellmittel (5) ausgestattet ist, Fixiermittel (8) und einen Abdominalabschnitt (6) umfasst, der über Verschlussmittel (16) verfügt;
- ein zweites Band (4), das einen Verbindungsabschnitt (7), der mit mindestens einem Haltungsrückstellmittel (5) ausgestattet ist, Fixiermittel (8) und einen Abdominalabschnitt (6) umfasst, der über komplementäre Verschlussmittel (17) verfügt, die dazu geeignet sind, auf einstellbare Weise mit den Verschlussmitteln (16) zusammenzuwirken; und
- eine Dorsalplatte (2), die Folgendes umfasst:
- komplementäre Fixiermittel (9), die zum Zusammenwirken mit den Fixiermitteln (8) des ersten und des zweiten Bands (3, 4) auf einstellbare Weise eingerichtet sind, und
- zwei Haltungsrückstellmittel (5), die dazu vorgesehen sind, auf beiden Seiten der Wirbelsäule eines Patienten positioniert zu werden;
wobei die Fixiermittel (8) des ersten und des zweiten Bands (3, 4) und die komplementären Fixiermittel (9) konform sind, um eine Positionierung von Haltungsrückstellmitteln (5) des ersten Bands (3) und des zweiten Bands (4) zu beiden Seiten von Haltungsrückstellmitteln (5) der Dorsalplatte (2) zuzulassen,
wobei die Orthese **dadurch gekennzeichnet ist, dass** das erste Band (3) eine Lasche (10) umfasst, die erste Einhakmittel (12) umfasst und dazu geeignet ist, zumindest zum Teil auf den Verbindungsabschnitt (7) des zweiten Bands (4), das komplementäre erste Einhakmittel (13) umfasst, gelegt zu werden, wobei die komplementären ersten Einhakmittel (13) dazu geeignet sind, auf einstellbare Weise mit den ersten Einhakmitteln (12) zusammenzuwirken, und dass das zweite Band (4) eine Lasche (10) umfasst, die komplementäre zweite Einhakmittel (15) umfasst und dazu geeignet ist, zumindest zum Teil auf den Verbindungsabschnitt (7) des ersten Bands (3), das zweite Einhakmittel (14) umfasst, gelegt zu werden, wobei die komplementären zweiten Einhakmittel (15) dazu geeignet sind, auf einstellbare Weise mit den zweiten Einhakmitteln (14) zusammenzuwirken.

2. Orthese (1) nach Anspruch 1, **dadurch gekennzeichnet, dass** die Abdominalabschnitte (6) des ersten und des zweiten Bands (3, 4) jeweils ein Haltungsrückstellmittel (5) umfassen.

3. Orthese (1) nach einem der Ansprüche 1 bis 2, **dadurch gekennzeichnet, dass** die Verbindungsabschnitte (7) des ersten und des zweiten Bands (3, 4) und der Dorsalplatte (2) Positionierungsreferenzen (18) umfassen, die zum Definieren von vorherbestimmten Einstellungspositionen eingerichtet sind, in denen die Haltungsrückstellmittel (5) des ersten und des zweiten Bands (3, 4) symmetrisch in Bezug auf die Haltungsrückstellmittel (5) der Dorsalplatte (2) positioniert sind.

4. Orthese (1) nach einem der Ansprüche 1 bis 3, **dadurch gekennzeichnet, dass** die Positionierungsreferenzen (18) Kontrastnähte sind, die auf der Dorsalplatte (2) und auf dem ersten und dem zweiten Band (3, 4) angeordnet sind.

5. Orthese (1) nach einem der Ansprüche 1 bis 4, **dadurch gekennzeichnet, dass** ein zentraler Teil der Dorsalplatte (2) oder die Gesamtheit der Dorsalplatte (2) eine axiale Abmessung umfasst, die größer als die des ersten und des zweiten Bands (3, 4) ist.

6. Orthese (1) nach einem der Ansprüche 1 bis 5, **dadurch gekennzeichnet, dass** die Fixiermittel (8) sich auf dem ersten und dem zweiten Band (3, 4) derart erstrecken, dass sie die Positionen von Haltungsrückstellmitteln (5) von Verbindungsabschnitten (7) des ersten und des zweiten Bands (3, 4) abdecken.

7. Orthese (1) nach einem der Ansprüche 1 bis 6, **dadurch gekennzeichnet, dass** die komplementären Fixiermittel (9) sich entlang der Längsrichtung der Orthese (1) erstrecken.

8. Orthese (1) nach einem der Ansprüche 1 bis 7, **dadurch gekennzeichnet, dass** das Haltungsrückstellmittel (5) von einem Stäbchen gebildet wird.

9. Orthese (1) nach einem der Ansprüche 1 bis 8, **dadurch gekennzeichnet, dass** die Fixiermittel (8), die komplementären Fixiermittel (9), die Einhakmittel (12, 14), die komplementären Einhakmittel (13, 15), die Verschlussmittel (16) und die komplementären Verschlussmittel (17) eine Textilie des Häkchen- oder Ösentyps umfassen.

10. Orthese (1) nach einem der Ansprüche 1 bis 9, **dadurch gekennzeichnet, dass** die Dorsalplatte (2), das erste und das zweite Band (3, 4) ein elastisches Textilmaterial umfassen.

## Claims

1. An orthosis (1) presenting the shape of a belt and including:
- a first strip (3) including a connecting portion (7) provided with at least a posture correcting means (5), fixing means (8) and an abdominal portion (6) having closing means (16);
- a second strip (4) including a connecting portion (7) provided with at least a posture correcting means (5), fixing means (8) and an abdominal portion (6) having complementary closing means (17) adapted to cooperate in adjustable manner with the closing means (16); and
- a back panel (2) including
o complementary fixing means (9) arranged to cooperate in adjustable manner with the fixing means (8) of the first and second strips (3,4), and
∘ two posture correcting means (5) intended to be positioned on either side of the vertebral column of a patient;
the fixing means (8) of the first and second strips (3,4) and the complementary fixing means (9) being shaped in such a way as to authorize a positioning of the posture correcting means (5) of the first strip (3) and the second strip (4) on either side of the posture correcting means (5) of the back panel (2),
the orthosis being **characterized in that** the first strip (3) comprises a tab (10) comprising first gripping means (12) and being able to be superimposed at least in part to the connecting portion (7) of the second strip (4) comprising first complementary gripping means (13), the first complementary gripping means (13) being able to cooperate in adjustable manner with the first gripping means (12), and **in that** the second strip (4) comprises a tab (10) comprising second complementary gripping means (15) and being able to be superimposed at least in part to the connecting portion (7) of the first strip (3) comprising second gripping means (14), the second complementary gripping means (15) being able to cooperate in adjustable manner with the second gripping means (14).

2. The orthosis (1) according to claim 1, **characterized in that** the abdominal portions (6) of the first and second strips (3, 4) include each a posture correcting means (5).

3. The orthosis (1) according to any of claims 1 to 2, **characterized in that** the connecting portions (7) of the first and second strips (3, 4) and the back panel (2) comprise positioning references (18) arranged to define predetermined adjustment positions in which the posture correcting means (5) of the first and second strips (3, 4) are symmetrically positioned with respect to the posture correcting means (5) of the back panel (2).

4. The orthosis (1) according to any of claims 1 to 3, **characterized in that** the positioning references (18) are contrasting seams disposed on the back panel (2) and on the first and second strips (3, 4).

5. The orthosis (1) according to any of claims 1 to 4, **characterized in that** a central part of the back panel (2) or the totality of the back panel (2) includes an axial dimension which is more significant than that of the first and second strips (3, 4).

6. The orthosis (1) according to any of claims 1 to 5, **characterized in that** the fixing means (8) extend over the first and second strips (3, 4) so as to cover the positions of the posture correcting means (5) of the connecting portions (7) of the first and second strips (3, 4).

7. The orthosis (1) according to any of claims 1 to 6, **characterized in that** the complementary fixing means (9) extend according to the longitudinal direction of the orthosis (1).

8. The orthosis (1) according to any of claims 1 to 7, **characterized in that** the posture correcting means (5) is formed by a stay.

9. The orthosis (1) according to any of claims 1 to 8, **characterized in that** the fixing means (8), the complementary fixing means (9), the gripping means (12, 14), the complementary gripping means (13, 15), the closing means (16) and the complementary closing means (17) include a hook-type or a loop-type textile.

10. The orthosis (1) according to any of claims 1 to 9, **characterized in that** the back panel (2), the first and second strips (3, 4) include an elastic textile material.
